# EUROPEAN PATENT APPLICATION

(11) **EP 1 022 028 A1**
(43) Date of publication of application: **26.07.2000**
(21) Application number: 98940557.6
(22) Date of filing: 26.08.1998
(51) Int. Cl.: A61K 45/00, A61K 47/30, A61K 31/70, A61K 9/16

(54) **SUSTAINED-RELEASE ORAL PREPARATION**

(30) Priority: 27.08.1997 JP 23054597
(71) Applicant: Takeda Chemical Industries, Ltd., Osaka-shi, Osaka 541-0045 (JP)
(72) Inventor: IGARI, Yasutaka, Kobe-shi, Hyogo 658-0015 (JP); AKIYAMA, Yohko, Ohmihachiman-shi, Shiga 523-0891 (JP); IWASAKI, Masato, Tokyo 112-0002 (JP)
(74) Representative: Lewin, John Harvey
(86) International application number: JP9803790
(87) International publication number: WO9910010

(57) **Abstract**

The sustained-release oral preparation containing a carbohydrolase inhibitor according to this invention sustainedly releases the carbohydrolase inhibitor, thus being effective in the prevention and treatment of diabetes through the inhibition of postprandial hyperglycemia, etc.

## Description

### TECHNICAL FIELD

The present invention relates to a sustained-release oral preparation which is useful as antidiabetics conducive to greater therapeutic benefits and improved compliance.

### BACKGROUND ART

As carbohydrolase inhibitors, known are α-glucosidase inhibitors represented by N-(1,3-dihydroxy-2-propyl)valiolamine, which is described in, inter alia, JP Kokai S57-200335, and acarbose, which is disclosed in JP Kokoku S54-39474, but pharmaceutical preparations containing them are generally available only in dosage forms to be administered at each mealtime.

Carbohydrolase inhibitors, particularly α -glucosidase inhibitors, competitively antagonize carbohydrolase in the gastrointestinal mucosa to inhibit or retard degradation of disaccharides to monosaccharides and thereby suppress postprandial elevation of the blood sugar level on oral administration, thus being antidiabetics of a novel type. However, it is essential that those inhibitors should be administered every mealtime. In order that they may express a sufficient inhibitory effect on hyperglycemia, carbohydrolase inhibitors need to come into contact with carbohydrloase in the digestive tract. Therefore, development of a new preparation is desired that will stay long in the digestive tract to enable sustained-release of the carbohydrolase inhibitor for enhanced therapeutic efficacy.

### DISCLOSURE OF INVENTION

In the above state of the art, the inventors of the present invention explored for a more effective, and safer pharmaceutical preparation which would prevent postprandial hyperglycemia, and found that the action of a carbohydrolase inhibitor is potentiated in a sustained-release oral preparation containing the inhibitor, particularly a prolonged gastrointestinal residence type preparation represented by a gastrointestinal mucosa-adherent preparation, an intragastric floating preparation, and an intragastric residence preparation utilizing a shape-memory type polymer. Further studies have led to completion of the present invention.

Namely, the present invention relates to:
(1) a sustained-release oral preparation comprising a carbohydrolase inhibitor;
(2) the preparation as defined in the above (1), wherein the sustained-release oral preparation is a prolonged gastrointestinal residence type preparation;
(3) the preparation as defined in the above (2), which is a prolonged upper digestive tract residence type preparation;
(4) the preparation as defined in the above (1), wherein the carbohydrolase inhibitor is an α-glucosidase inhibitor;
(5) the preparation as defined in the above (1), which is gastrointestinal mucosa-adherent;
(6) the preparation as defined in the above (1), which further comprises a matrix comprising a polyglycerol fatty acid ester and/or a lipid;
(7) the preparation as defined in the above (6), which further comprises a substance which develops viscosity in response to water;
(8) the preparation as defined in the above (6), wherein the carbohydrolase inhibitor is contained in the matrix;
(9) the preparation as defined in the above (7), wherein the substance which develops viscosity in response to water is contained in the matrix;
(10) the preparation as defined in the above (7), wherein the matrix is coated with a substance which develops viscosity in response to water;
(11) the preparation as defined in the above (1), wherein the carbohydrolase inhibitor is voglibose;
(12) the preparation as defined in the above (1), which is for antidiabetics;
(13) the preparation as defined in the above (1), which is a solid preparation;
(14) the preparation as defined in the above (13), wherein the solid preparation is in the form of fine granules or granules;
(15) the preparation as defined in the above (1), wherein the carbohydrolase inhibitor is contained in a proportion of about 0.05 ∼ about 5.0 weight % based on the whole preparation;
(16) the preparation as defined in the above (6), wherein the polyglycerol fatty acid ester has a molecular weight of about 200 ∼ about 5000;
(17) the preparation as defined in the above (6), wherein the polyglycerol fatty acid ester has a hydrophile-lipophile balance number of 1∼15;
(18) the preparation as defined in the above (6), wherein the polyglycerol fatty acid ester is used in a proportion of about 0.01 ∼ about 10000 weight parts per weight part of the carbohydrolase inhibitor;
(19) the preparation as defined in the above (7), wherein the substance which develops viscosity in response to water is an acrylic acid polymer or a salt thereof; and
(20) the preparation as defined in the above (7), wherein the substance which develops viscosity in response to water is contained in a proportion of about 0.05 ∼ about 99 weight % based on the whole matrix.

### BEST MODE FOR CARRYING OUT THE INVENTION

The carbohydrolase inhibitor for use in the present invention includes amylase inhibitors and α-glucosidase inhibitors, among others, preferably α-glucosidase inhibitors. As examples of the α-glucosidase inhibitor, there can be mentioned valiolamine derivatives of the general formula: 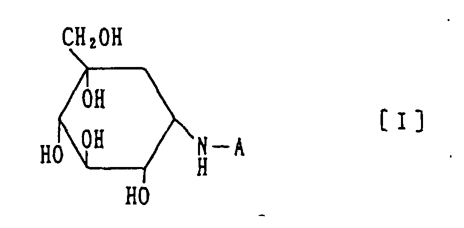 [wherein A represents a C₁₋₁₀ acyclic hydrocarbon group optionally having hydroxyl, phenoxy, thienyl, furyl, pyridyl, cyclohexyl, or optionally substituted phenyl; a 5- or 6-membered cyclic hydrocarbon group optionally having hydroxyl, hydroxymethyl, methyl or amino; or a sugar residue] which are described in the specifications of JP Kokai S57-200335, JP Kokai S58-59946, JP Kokai S58-162597, JP Kokai S58-216145, JP Kokai S59-73549, JP Kokai S59-95297, and so forth.

Referring to the above general formula [I], A may for example be a C₁₋₁₀ straight-chain or branched-chain saturated or unsaturated aliphatic hydrocarbon group which may have such substituents as hydroxyl, phenoxy, thenyl, furyl, pyridyl, cyclohexyl, optionally substituted phenyl, etc. Examples of the substituent in said optionally substituted phenyl include lower(C₁₋₆)alkyl, lower(C₁₋₆) alkoxy, halogen (e.g. chloro, bromo, fluoro, iodo) and phenyl, among others.

Further examples of A include 5- or 6-membered cyclic hydrocarbon groups and sugar residues. These groups may have such substituents as hydroxyl, hydroxymethyl, methyl and amino. The sugar residue means the group which remains after removal of one hydrogen atom from a sugar molecule and includes sugar residues derived from monosaccharides and oligosaccharides, for instance.

Each of those derivatives may be in the form of the salt with an inorganic acid such as hydrochloric acid, or an organic acid such as citric acid.

Specific examples of the N-substituted valiolamine derivative of general formula [I] include:
(1) N-phenethylvaliolamine;
(2) N-(3-phenylallyl)valiolamine;
(3) N-furfurylvaliolamine;
(4) N-thenylvaliolamine;
(5) N-(3-pyridylmethyl)valiolamine;
(6) N-(4-bromobenzyl)valiolamine;
(7) N-[(R)-β-hydroxyphenethyl]valiolamine;
(8) N-[(S)-β-hydroxyphenethyl]valiolamine;
(9) N-(α-hydroxy-2-methoxyphenethyl)valiolamine;
(10) N-(3,5-di-tert-butyl-4-hydroxybenzyl)valiolamine;
(11) N-(cyclohexylmethyl)valiolamine;
(12) N-geranylvaliolamine;
(13) N-(1,3-dihydroxy-2-propyl)valiolamine;
(14) N-(1,3-dihydroxy-1-phenyl-2-propyl)valiolamine;
(15) N-[(R)-α-(hydroxymethyl)benzyl]valiolamine;
(16) N-cyclohexylvaliolamine;
(17) N-(2-hydroxycyclohexyl)valiolamine;
(18) N-[(1R,2R)-2-hydroxycyclohexyl]valiolamine;
(19) N-(2-hydroxycyclopentyl)valiolamine;
(20) methyl 4-[(1S,2S)-(2,4,5(OH)/3,5)-2,3,4,5-tetrahydroxy-5-(hydroxymethyl)cyclohexyl]-amino-4,6-dideoxy-α-D-glucopyranoside;
(21) methyl 4-[(1S,2S)-(2,4,5(OH)/3,5)-2,3,4,5-tetrahydroxy-5-(hydroxymethyl)cyclohexyl]-amino-4-deoxy-α-D-glucopyranoside;
(22) [(1S,2S)-(2,4,5(OH)/3,5)-2,3,4,5-tetrahydroxy-5-(hydroxymethyl)cyclohexyl][(1R,2S)-(2,6/3,4)-4-amino-2,3-dihydroxy-6-(hydroxymethyl)cyclohexyl]amine;
(23) N-[(1R,2S)-(2,4/3,5)-2,3,4-trihydroxy-5-(hydroxymethyl)cyclohexyl]valiolamine,
(24) N-[(1R,2S)-(2,6/3,4)-4-amino-2,3-dihydroxy-6-methylcyclohexyl]valiolamine;
(25) N-[(1R,2S)-(2.6/3,4)-2,3,4-trihydroxy-6-methylcyclohexyl]valiolamine,
(26) N-[(1R,2S)-(2,4,6/3)-2,3,4-trihydroxy-6-methylcyclohexyl]valiolamine,
(27) 4-O-α-[4-[((1S)-(1,2,4,5(OH)/3,5)-2,3,4,5-tetrahydroxy-5-(hydroxymethyl)cyclohexyl)amino]-4,6-dideoxy-D-glucopyranosyl]-D-glucopyranose; and
(28) 1,6-anhydro-4-O-α-[4-[((1S)-(1,2,4,5(OH)/3,5)2,3,4,5-tetrahydroxy-5-C-(hydroxymethyl)cyclohexyl)amino]-4,6-dideoxy-D-glucopyranosyl]-α-D-glucopyranose.

Particularly preferred, among these, is N-(1,3-dihydroxy-2-propyl)valiolamine, i.e. [2-hydroxy-1-(hydroxymethyl)ethyl]valiolamine or 1L(1S)-(1(OH)2,3,5/1,3)-5-[[2-hydroxy-1-(hydroxymethyl)ethyl]amino]-1-C-(hydroxymethyl)-1,2,3,4-cyclohexanetetrol(hereinafter sometimes referred to as voglibose).

Further α-glucosidase inhibitors which can be used preferably are N-substituted derivatives of valienamine of the general formula: 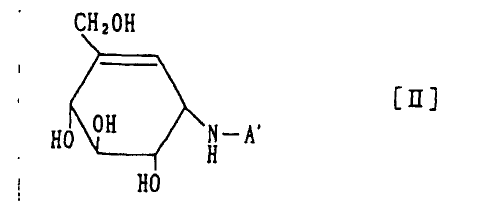 [wherein A' represnts a C₁₋₁₀ acyclic hydrocarbon group which may be substituted by hydroxyl, phenoxy, thienyl, furyl, pyridyl, cyclohexyl, optionally substituted phenyl (examples of the substituent include the same substituents as defined for A above, such as lower (e.g. C₁₋₆)alkyl, lower (e.g. C₁₋₆) alkoxy, halogen, phenyl and so on); a 5- or 6-membered cyclic hydrocarbon group which may be substituted by hydroxyl, hydroxymethyl, methyl or amino; or a sugar residue] which are described in JP Kokal S57-64648 etc. and
N-substituted derivatives of validamine of the general formula: 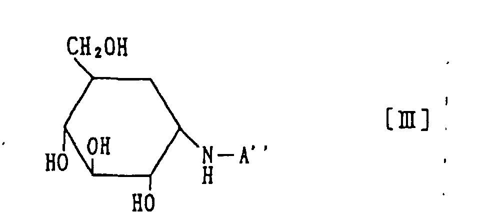 [wherein A'' represents a C₁₋₁₀ acyclic hydrocarbon group which may be substituted by hydroxyl, phenoxy, thienyl, furyl, pyridyl, cyclohexyl, or phenyl optionally substituted (by the same substituents as defined for A or A' above); a 5- or 6-membered cyclic hydrocarbon group which may be substituted by hydroxyl, hydroxymethyl, methyl or amino; or a sugar residue] which are described in JP Kokai S57-114554.

Furthermore, the following compounds can also be used as the α-glucosidase inhibitor: acarbose (BAY g 5421, Naturwissenschaften 64, 535-537, 1997, JP Kokoku S54-39474); trestatin (J. Antibiotics, 36, 1157-1175, 1983 and 37, 182-186, 1984 and JP Kokai S54-163511); adiposins (J. Antibiotics, 35, 1234-1236, 1982, J. Jap. Soc. Starch Sci, 26, 134-144, 1979 & 27, 107-113, 1980, JP Kokai S54-106402; S54-106403; S55-64509; S56-123986 and S56-125398); amylostatins (Agric. Biol. Chem., 46, 1941-1945, 1982, JP Kokai S50-123891; S55-71494; S55-157595), oligostatins (SF-1130X, JP Kokai S53-26398 & S56-43294 and J. Antibiotics, 34, 1424-1433, 1981); and aminosugar compounds (JP Kokai S54-92909). Regarding α-glucosidase inhibitors of the microbial origin, inclusive of the above-mentioned compounds, a general review by E. Truscheit (Angewandte Chemie, 93, 738-755, 1981) are reported.

Furthermore, the compounds available on methanolysis of acarbose or oligostatin C, namely methyl 4-[(1S,6S)-(4,6/5)-4,5,6-trihydroxy-3-hydroxymethyl-2-cyclohexen-1-yl]amino-4,6-dideoxy-α-D-glucopyranoside [182nd ACS National Meeting Abstracts Paper, MEDI 69, August 1981, New York, J. Antibiotics, 34, 1429-1433, 1981, and JP Kokai S57-24397], 1-deoxynojirimycin (Naturwissenschaften, 66, 584-585, 1979) and N-substituted derivatives thereof, e.g. BAY m 1099 and BAY o 1248 (J. Clin. Invest. 14 (2-II), 47, 1984; Diabetologia, 27 (2), 288A, 346A and 323A, 1984) can also be used as the α-glucosidase inhibitor.

These carbohydrolase inhibitors can be used in a suitable combination of two or more species.

In the sustained-release oral preparation of the present invention, the carbohydrolase inhibitor is contained in a proportion of generally about 0.01 ∼ about 50% (w/w), preferably about 0.05 ∼ about 5.0 % (w/w), more preferably about 0.1 ∼ about 1.0%, based on the whole preparation, although the proportion may vary depending on other ingredients used.

The sustained-release oral preparation of the present invention can be provided in any form that is capable of controlling release of the carbohydrolase inhibitor after administration. Particularly preferred, among such controlled-release preparation, are those suited for oral administration. Especially preferred are those controlling release of carbohydrolase inhibitor, exemplified by a diffusion type such as a matrix type and a swellable type.

The sustained-release oral preparation of the present invention is preferably a prolonged gastrointestinal residence type preparation which either attaches itself to the gastrointestinal mucosa or float or swell in the digestive tract to thereby stay long. Furthermore, the sustained-release oral preparation of the present invention may be any preparation adapted to prolong the residence time of the carbohydrolase inhibitor in the digestive tract by sustained-release at a suitable rate or discontinuously or intermittently release or release a carbohydrolase inhibitor over a long time in response to a dietary stimulation, to establish contact between the carbohydrolase inhibitor and the carbohydrolase for the expression and potentiation of therapeutic efficacy. The intragastrointestinal residence time is preferably about a few hours to about 20 hours, and at least about 3 ∼ about 4 hours (preferably about 4 ∼ about 5 hours) to about 12 hours. The site of residence is preferably, among the digestive tract, the region of the digestive tract where the carbohydrolase are present or the upper digestive tract. It is particularly preferable that the preparation stays long in the region of the digestive tract extending from the stomach to the duodenum and the upper part of the small intestine for assuring the contact of the inhibitor with the carbohydrolase.

Preferred examples of the prolonged gastrointestinal residence type preparation include a preparation containing a matrix comprising a polyglycerol fatty acid ester and/or a lipid. To provide a gastrointestinal mucosa-adherent type preparation, it is still more preferable to incorporate a prolonged gastrointestinal residence type matrix containing a substance which develops viscosity in response to water (hereinafter sometimes referred to briefly as viscogenic agent), for example a prolonged gastrointestinal residence type matrix comprising a polyglycerol fatty acid ester and/or a lipid and a viscogenic agent is preferred.

The prolonged gastrointestinal residence type matrix is preferably such that the viscogenic agent is dispersed in a matrix comprising a polyglycerol fatty acid ester and/or a lipid or such a matrix coated with the viscogenic agent.

The melting point of the prolonged gastrointestinal residence type matrix is for example about 30 ∼ about 120°C, preferably about 40 ∼ about 120° C.

Polyglycerol fatty acid esters may be monoesters or polyesters such as diesters and triesters, as long as they are esters of polyglycerols with fatty acids. The polyglycerol fatty acid esters show no crystal polymorphism and are characterized in that they hardly interact with the carbohydrolase inhibitor. Therefore, the carbohydrolase inhibitor in the presence of a polyglycerol fatty acid ester is little deactivated and remains stable for a long time.

Polyglycerol is a "polyhydric alcohol containing, in each molecule, n (cyclic form) to n+2 (straight or branched form) hydroxyl groups and n-1 (straight or branched form) to n (cyclic form) ether bonds" ["Polyglycerin Ester", edited by Sakamoto Yakuhin Kogyo Co., Ltd., October 4, 1994] and may be straight-chain or branched. As the polyglycerol, there can be employed compounds of the formula : 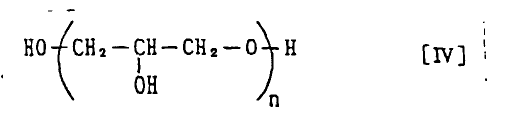 (wherein n represents the degree of polymerization which is an integer of not less than 2).

In the above formula, n is generally 2 to 50, preferably 2 to 20, and, more preferably 2 to 10. Typical examples of such polyglycerols are diglycerol, triglycerol, tetraglycerol, pentaglycerol, hexaglycerol, heptaglycerol, octaglycerol, nonaglycerol, decaglycerol, pentadecaglycerol, eicosagiycerol and triacontaglycerol. Among these polyglycerols, tetraglycerol, hexaglycerol and decaglycerol are used most frequently.

The fatty acid typically includes saturated or unsaturated fatty acids containing 8 to 40 carbon atoms, preferably 12 to 22 carbon atoms. The preferred fatty acids include stearic acid, oleic acid, lauric acid, linolic acid and behenic acid.

As specific examples of such polyglycerol fatty acid esters, there may be mentioned behenyl hexa(tetra)glyceride, caprylyl mono(deca)glyceride, caprylyl di(tri)glyceride, capryl di(tri)glyceride, lauryl mono(tetra)glyceride, lauryl mono(hexa)glyceride, lauryl mono(deca)glyceride, oleyl mono(tetra)glyceride, oleyl mono(hexa)glyceride, oleyl mono(deca)glyceride, oleyl di(tri)glyceride, oleyl di(tetra)glyceride, oleyl sesqui(deca)glyceride, oleyl penta(tetra)glyceride, oleyl penta(hexa)glyceride, oleyl deca(deca)glyceride, linolyl mono(hepta)glyceride, linoyl di(tri)glyceride, linoyl di(tri)glyceride, linoyl di(tetra)glyceride, linolyl di(hexa)glyceride, stearyl mono(di)glyceride, stearyl mono(tetra)glyceride, stearyl penta(tetra)glyceride, stearyl mono(deca)glyceride, stearyl tri(tetra)glyceride, stearyl penta(hexa)glyceride, stearyl tri(hexa)glyceride, stearyl deca(deca)glyceride, palmityl mono(tetra)glyceride, palmityl mono(hexa)glyceride, palmityl mono(deca)glyceride, palmityl tri(tetra)glyceride, palmityl tri(hexa)glyceride, palmityl sesqui(hexa)glyceride, palmityl penta(tetra)glyceride, palmityl penta(hexa)glyceride and palmityl deca(deca)glyceride.

Preferred polyglycerol fatty acid esters include, for example, behenyl hexa(tetra)glyceride (e.g. produced by Sakamoto Yakuhin Kogyo Co., Trade name : HB-310; produced by Riken Vitamin Co., Ltd., Trade name: Poem J-46B, etc.), stearyl penta(tetra)glyceride (e.g. produced by Sakamoto Yakuhin Kogyo Co., Ltd., Trade name : PS-310), stearyl mono(tetra)glyceride (e.g. produced by Sakamoto Yakuhin Kogyo Co., Ltd., Trade name: MS-310), stearyl penta(hexa)glyceride (e.g. produced by Sakamoto Yakuhin Kogyo Co., Ltd., Trade name PS-500), stearyl mono(deca)glyceride, and mixtures thereof.

The above polyglycerol fatty acid esters may be used in combination of two or more species, preferably two or three species.

The molecular weight of the polyglycerol fatty acid ester is generally about 200 to about 5000, preferably about 300 to about 2000, more preferably about 500 to about 2000. The HLB (hydrophile-lipophile balance) number of the polyglycerol fatty acid ester is generally 1 to 22, preferably 1 to 15, more preferably about 1 to 9, still more preferably about 2∼9. The HLB number may be adjusted by admixing two or more polyglycerol fatty acid esters having different HLB numbers. By varying the HLB number of the polyglycerol fatty acid ester, the release and dissolution rates of the carbohydrolase inhibitor can be controlled.

While polyglycerol fatty acid esters can be selectively used according to the kinds of carbohydrolase inhibitor, viscogenic agent used concomitantly, and matrix form, those which are solid at ambient temperature (about 15°C) are preferred. The melting point of the polyglycerol fatty acid ester is, for example, about 15°C to about 80°C, preferably about 30°C to about 75°C, more preferably about 45°C to about 75°C.

When two or more polyglycerol fatty acid esters are used as a mixture, liquid polyglycerol fatty acid esters may be used concomitantly as long as the prolonged gastrointestinal residence type matrix is solid at ambient temperature.

When the polyglycerol fatty acid ester is used for the prolonged gastrointestinal residence type matrix, the amount of the polyglycerol fatty acid ester used may for example be about 0.01 ∼ about 10000 parts by weight, preferably about 0.1 ∼ about 2000 parts by weight, for each part by weight of the carbohydrolase inhibitor in the sustained-release oral preparation.

As the lipid, used is one having a melting point of about 40 to about 120°C, preferably about 40 to about 90°C.

The lipid includes saturated fatty acids having 14 to 22 carbon atoms (e.g. myristic acid, stearic acid, palmitic acid, behenic acid, etc.) and salts thereof (e.g. sodium salts, potassium salts); higher alcohols of 16∼22 carbon atoms (e.g. cetyl alcohol, stearyl alcohol, etc.); fatty acid glycerol esters such as the monoglycerides, diglycerides and triglycerides of said fatty acids (e.g. 1-monostearin, 1-monopalmitin, etc.); oils and fats (e.g. castor oil, cottonseed oil, beef tallow, etc.) and hydrogenated versions thereof; waxes (e.g. beeswax, carnauba wax, sperm wax, etc.); hydrocarbons (e.g. paraffin, microcrystalline, wax, etc.); and phospholipids (e.g. hydrogenated lecithin), among others. Among these lipids, the lipids preferred from the standpoint of adhesiveness includes saturated fatty acids of 14∼22 carbon atoms, higher alcohols of 16∼22 carbon atoms, oils and fats or hydrogenated versions thereof, waxes and hydrocarbons. More particularly, hydrogenated cottonseed oil, hydrogenated castor oil, hydrogenated soybean oil, carnauba wax, stearic acid, stearyl alcohol, microcrystalline wax, etc. are preferred. The particularly preferred lipid is hydrogenated castor oil or carnauba wax. In terms of the continuous or sustained release of the carbohydrolase inhibitor, hydrogenated castor oil, carnauba wax and microcrystalline wax are particularly preferred.

When a lipid is used for the prolonged gastrointestinal residence type matrix, the amount of the lipid used is about 0.01 ∼ about 10000 parts, preferably about 0.1 ∼ about 1000 parts, per part of the carbohydrolase inhibitor.

The polyglycerol fatty acid ester and lipid mentioned above may be used as mixed in advance; for example, a mixture of a polyglycerol fatty acid ester with a wax, or a mixture of a polyglycerol fatty acid ester with a hydrogenated oil can be used. More particularly, a mixture of one or more members selected from among behenyl hexa(tetra)glyceride, stearyl penta(tetra)glyceride and stearyl penta(hexa)glyceride with one or more members selected from among carnauba wax, hydrogenated castor oil and microcrystalline wax can be employed.

The substance which develops viscosity in response to water (viscogenic agent) is not particularly restricted insofar as it shows a viscosity build-up in the presence of water to adhere to the gastrointestinal mucosa and is pharmaceutically acceptable. Among such substances, those which swell remarkably in the presence of water to develop considerable viscosity are preferred. Such viscogenic agents may for example be polymers and naturally-occurring mucous substances. Suitable polymers are such that the viscosity of a 2% aqueous polymer solution at 20°C is about 3 ∼ about 50000 cps, preferably about 10 ∼ about 30000 cps, more preferably about 3 ∼ about 30000 cps. However, as far as a polymer that is increased in viscosity on neutralization is concerned, the viscosity of a neutralized 0.2% solution of the polymer at 20°C is about 100 ∼ about 500000 cps, preferably about 100 ∼ about 200000 cps, more preferably about 1500 ∼ about 1000000 cps.

The polymer mentioned above is preferably an acidic polymer and, as examples thereof, polymers having a carboxyl or sulfo group, or a salt thereof, can be mentioned. Particularly preferred are polymers having a carboxyl group or a salt thereof.

The polymer having a carboxyl group or a salt thereof includes but is not limited to acrylic acid polymers (inclusive of copolymers) comprising acrylic acid as a constituent monomer unit and their salts. Said salt includes monovalent metal salts such as the sodium salt, potassium salt, etc.; and divalent metal salts such as the magnesium salt and calcium salt. The acrylic acid polymer or its salt includes polymers containing about 58 ∼ about 63 weight % of carboxyl groups and having molecular weights of about 20×10⁴ ∼ about 600×10⁴, preferably about 100×10⁴ ∼ about 600×10⁴, more preferably about 100×10⁴ ∼ about 500×10⁴. The preferred acrylic acid polymer or salt includes acrylic acid homopolymers and salts thereof. Furthermore, the acrylic acid polymer is preferably a crosslinked acrylic acid polymer.

Such polymers are listed under the heading of Carboxyvinyl Polymers in the Formulary of Non-Official Drugs, Japan (October, 1986). As specific examples of such polymers, there can mentioned carbomers (Trade name: Carbopol, The B.F. Goodrich Company, 940, 934, 934P, 941, 1342, 974P (NF XVIII), etc.; HIVISWAKO 103, 104, 105 (Wako Pure Chemical Ind. Co.); Noveon AA1 (The B.F. Goodrich Company); and calcium polycarbophil (USP XXIII), among others.

The naturally-occurring mucous substance includes curdlan (Curdlan N, a food additive) which is a water-insoluble linear polysaccharide (β -1,3-glucan) produced by microorganisms (Alcaligenes faecalis var myxogenes), mucin, agar, gelatin, pectin, carrageenan, sodium alginate, locust bean gum, xanthan gum, tragacanth gum, chitosan, pullulan, waxy starch, sucralfate, cellulose and its derivatives (e.g. cellulose sulfate etc.), and so on. The preferred are hydroxypropylcellulose and hydroxypropylmethylcellulose.

The viscogenic agent for use in the present invention is preferably an acrylic acid polymer or its salt.

Those viscogenic agents can be used in a suitable combination of two or more species.

The amount of the viscogenic agent used may for example be about 0.005 ∼ about 99 weight %, preferably about 0.5 ∼ about 45 weight %, more preferably about 5 ∼ about 30 weight %, based on the whole prolonged gastrointestinal residence type matrix. When, for example, the viscogenic agent is dispersed in a matrix comprising a polyglycerol fatty acid ester and/or a lipid, the amount of the viscogenic agent used is about 0.005 ∼ about 95 weight %, preferably about 0.5 ∼ about 30 weight %, more preferably about 5 ∼ about 25 weight %, based on the whole matrix. When the matrix is coated with the viscogenic agent, the amount of the viscogenic agent used is about 0.005 ∼ about 95 weight %, preferably about 0.5 ∼ about 30 weight %, more preferably about 5 ∼ about 25 weight %, based on the whole matrix.

Since carbohydrolase inhibitors in general are readily soluble in water, it is difficult to provide a carbohydrolase inhibitor-containing preparation showing a satisfactory release profile. However, use of a viscogenic agent enables production of a preparation capable of releasing a carbohydrolase inhibitor continuously over a long time.

When, for example, the prolonged gastrointestinal residence type matrix used is a prolonged gastrointestinal residence type matrix wherein a viscogenic agent is incorporated in a polyglycerol fatty acid ester, or a prolonged gastrointestinal residence type matrix wherein a viscogenic agent is incorporated in a lipid, the amount of the polyglycerol fatty acid ester or lipid is about 0.01 ∼ about 10000 parts by weight, preferably about 0.1 ∼ about 1000 parts by weight, relative to each part by weight of the carbohydrolase inhibitor in the sustained-release oral preparation.

Furthermore, a lipid may be additionally incorporated in the above matrix comprising a polyglycerol fatty acid ester. The lipid which can be used is a pharmaceutically acceptable water-insoluble substance having the property to modulate the rate of release of the carbohydrolase inhibitor. As such lipid, the same lipids as mentioned hereinbefore, for instance, can be employed.

When a lipid and a polyglycerol fatty acid ester are used in combination, the amount of the lipid and the polyglycerol fatty acid ester used may be respectively selected within the range not detracting from the adhesion to the gastrointestinal mucosa; thus, the amount of the polyglycerol fatty acid ester based on the carbohydrolase inhibitor in the sustained-release oral preparation may for example be about 0.01 ∼ about 10000 parts by weight, preferably about 0.1 ∼ about 1000 parts by weight, and the amount of the lipid relative to the polyglycerol fatty acid ester may for example be about 0.01 ∼ about 10000 parts by weight, preferably about 0.1 ∼ about 2000 parts by weight, more preferably about 0.1 ∼ about 1000 parts by weight.

The prolonged gastrointestinal residence type preparation in which a viscogenic agent is dispersed in a matrix comprising said polyglycerol fatty acid ester and/or lipid may be such that the viscogenic agent and the active ingredient carbohydrolase inhibitor are dispersed in said polyglycerol fatty acid ester and/or lipid. As the method for dispersion, per se known method is employed.

The sustained-release oral preparation of the present invention, particularly the prolonged gastrointestinal residence type preparation, may contain an organic acid in a suitable proportion for the purpose of promoting absorption of the carbohydrolase inhibitor. The organic acid that can be used includes for example tartaric acid, citric acid, succinic acid and ascorbic acid.

When the sustained-release oral preparation is a solid preparation, it may contain those additives which are generally employed in the manufacture of solid pharmaceutical preparations (e.g. tablets, fine granules, granules, etc.). Such additives include various excipients such as corn starch, talc, crystalline cellulose (Avicel, powdered sugar, magnesium stearate, mannitol, light silicic anhydride, magnesium carbonate, calcium carbonate, L-cysteine, lactose, etc.; binders such as starch, sucrose, gelatin, gum arabic powder, methylcellulose, carboxymethylcellulose, carboxymethylcellulose sodium, hydroxypropylcellulose, hydroxypropylmethylcellulose, polyvinylpyrrolidone, pullulan, dextrin, etc.; disintegrators such as carboxymethylcellulose calcium, low-substituted hydroxypropylcellulose, croscarmellose sodium, etc.; anionic surfactants such as sodium alkyl sulfates etc.; nonionic surfactants such as polyoxyethylene-sorbitan fatty acid esters, polyoxyethylene-fatty acid esters, polyoxyethylene-castor oil derivatives, etc.; antacids or mucosal protectants such as magnesium hydroxide, magnesium aluminunate silicate, sucralfate, etc.; lubricants such as magnesium stearate, talc, etc.; coloring agents; corrigents; adsorbents; antiseptics; parting agents; antistatic agents; and so on. The amount of these additives added can be selected within the range not detracting from the adhesion of the preparation to the mucosa.

The sustained-release oral preparation according to the present invention may be a solid preparation or a liquid preparation. Preferably, the sustained-release oral preparation is a solid preparation. The dosage form of such a solid preparation includes fine granules; granules; tablets; pills; tablets obtainable by admixing said fine granules or granules with the above excipient, binder, disintegrant and lubricant and tabletting the mixture; and capsules obtainable by filling capsule shells with the above fine granules or granules. Preferred, among them, are fine granules and granules.

The particle size distribution of fine granules may for example be such that at least 75 weight % of particles are 10 to 500 µ m in diameter, at most 5 weight % of particles are not smaller than 500 µ m, and at most 10 weight % of particles are not larger than 10 µ m. The preferred particle size distribution of fine granules is such that at least 75 weight % of particles are 105 to 500 µ m in diameter, at most 5 weight % of particles are not smaller than 500 µ m, and at most 10 weight % of particles are not larger than 74 µ m. The particle size distribution of granules may for example be such that at least 90 weight % of particles are 500∼1410 µ m and at most 5 weight % of particles are not larger than 177 µ m in diameter.

The dosage form of a liquid preparation includes a syrup; an emulsion; a suspension; and a capsule obtainable by filling capsule shells with syrup, emulsion or suspension; among others.

The sustained-release oral preparation of the present invention can be manufactured by a per se known method and conventional techniques. Typical processes for manufacturing prolonged gastrointestinal residence type preparations, for instance, are described below.
1) When the prolonged gasrointestinal residence type preparation is solid at ambient temperature, it can be manufactured by a per se known method. For example, there can be mentioned a process which comprises heating and melting the polyglycerol fatty acid ester and/or lipid to its melting point or a higher temperature, adding the viscogenic agent and the carbohydrolase inhibitor to the resulting melt simultaneously or separately for dispersion, and cooling the resulting dispersion. The heating temperature is for example about 40°C ∼ about 150°C, preferably about 50°C ∼ about 110°C, more preferably about 50°C ∼ about 90°C. This process can be carried out using a conventional granulating machine or the like. For instance, a spray-cooling technique such as spray-chilling is preferably employed to provide spherical solid preparations (e.g. fine granules). The spray-chilling can be carried out by, for example, the method which comprises dripping a dispersion of the viscogenic agent and carbohydrolase inhibitor in the molten polyglycerol fatty acid ester and/or lipid at a constant rate on a rotary disk revolving at a high speed of, for example, 10∼6000 revolutions per minute, preferably 900∼6000 rpm, more preferably 1000∼3000 rpm. The rotary disk that can be used may be a flat smooth disk, for example an aluminum disk of 5∼100 cm in diameter, preferably 10∼20 cm in diameter. The rate of dripping the molten dispersion can be selected according to the desired particle diameter of the product and is generally about 2 g ∼ about 200 g/minute, preferably about 5 g ∼ about 100 g/minute. The granules thus obtained are almost true spheres so that, in the subsequent coating step, a uniform coating layer can be formed with high efficiency.
   An alternative process which can be employed comprises dispersing and granulating the viscogenic agent and carbohydrolase inhibitor in the polyglycerol fatty acid ester and/or lipid by, for example, a kneading technique. The solvent for use in this process may be the conventional solvent (e.g. methanol, acetonitrile, chloroform, etc.).
   Furthermore, a solid preparation can be manufactured by the melt-granulating technology. The melt-granulating technology may for example be the process which comprises heating the polyglycerol fatty acid ester and/or lipid in the vicinity of its melting point, for example within the range from the melting point to a temperature lower than said melting point by about 5°C, subjecting the resulting melt to a granulation process such as the above spray-chilling, to prepare fine granules, and floating them together with the viscogenic agent and carbohydrolase inhibitor while heating at a suitable elevated temperature to provide a prolonged gastrointestinal residence type matrix. In this case, the effect of heat on the active ingredient carbohydrolase inhibitor can be prevented so that a matrix can be easily obtained avoiding deactivation of the active ingredient.
   The sustained-release oral preparation of the present invention may be coated with a coating agent. In the case of a solid preparation such that a matrix comprising a polyglycerol fatty acid ester and/or lipid is coated with a viscogenic agent, for instance, said matrix need only be coated with a coating agent containing said viscogenic agent. The coating agent may contain at least one ingredient selected from among the above polyglycerol fatty acid ester, the above lipid and a water-insoluble polymer such as that described below. When, in this case, a viscogenic agent which is poorly compatible or incompatible with ingredients in the solid preparation, the matrix can be coated with a film wherein the viscogenic agent is dispersed. Furthermore, the coating agent may contain various additives which are commonly used in solid pharmaceutical preparations.
   Examples of the water-insoluble polymer includes hydroxypropylmethylcellulose phthalate (Japanese Pharmacopoeia [JP] XII), hydroxypropylmethylcellulose acetate succinate (produced by Shin-Etsu Chemical), carboxymethylethylcellulose (Freund Industrial Co., Ltd., CMEC, the Formulary of Non-official Drugs 1986), cellulose acetate trimellitate (produced by Eastman), cellulose acetate phthalate (JP XII), ethylcellulose (produced by Asahi Chemical Industry), aminoalkyl methacrylate copolymer (produced by Rohm Pharma, Trade name : Eudragit RS-100, RL-100, RL-PO, RS-PO, RS-30D, RL-30D), methacrylic acid/ethyl acrylate copolymer (produced by Rohm Pharma, Trade name : Eudragit L100-55), methacrylic acid/methyl methacrylate copolymer (produced by Rohm Pharma, Trade name : Eudragit L100, S-100), Eudragit L-30D-55, Eudragit NE-30D (produced by Rohm Pharma), and polyvinyl acetate (produced by Colorcon). Those water-insoluble polymers may be used in a suitable combination of two or more species.
   The amount of the viscogenic agent used in the coating agent is about 0.005 ∼ about 100 weight %, preferably about 0.05 ∼ about 95 weight %, more preferably about 1 ∼ about 10 weight %, based on the total solid fraction of the coating agent.
   When at least one member selected from among the polyglycerol fatty acid ester, lipid and water-insoluble polymer is used in combination with the viscogenic agent as the coating agent, the amount of the viscogenic agent used is about 0.05 ∼ about 95 weight %, preferably about 0.5 ∼ about 95 weight %, more preferably about 5 ∼ about 30 weight %, based on the total solid fraction of the coating agent.
   Furthermore, two or more members selected from among the polyglycerol fatty acid ester, lipid and water-insoluble polymer can be used in combination in the coating agent. In this case, the amount of the other member used is about 0.0001 ∼ about 1000 weight %, preferably about 0.01 ∼ about 100 weight %, more preferably about 0.01 ∼ about 10 weight %, relative to each unit weight of the total polyglycerol fatty acid ester and/or lipid.
   The coating amount of the coating agent can be selected with reference to the kind of composition to be coated such as matrix, and the desired adhesiveness to the mucosa, among other conditions. The coating amount relative to the composition to be coated may for example be about 0.1 ∼ about 30 weight %, preferably about 0.5 ∼ about 20 weight %, for tablets; and about 0.1 ∼ about 100 weight %, preferably about 1 ∼ about 50 weight %, for fine granules.
   In the coating procedure, coating may be carried out by using a coating agent to which the above conventional additives have been added in advance or using independently the coating agent and the above additives. The amount of the additives, based on the solid fraction of the coating agent, is about 0.1 ∼ about 70 weight %, preferably about 1 ∼ about 50 weight %, more preferably about 20 ∼ about 50 weight %.
   As the coating techniques, per se known methods such as the pan coating method, the fluidized-bed coating method, or the centrifugal coating method can be employed. When the coating agent is a solution or dispersion containing water or an organic solvent, the spray-coating method can also be employed. The kind of said water or organic solvent is not particularly restricted but includes alcohols such as methanol, ethanol, isopropyl alcohol, and etc.; ketones such as acetone and etc.; and halogenated hydrocarbons such as chloroform, dichloromethane, trichloromethane and etc.
   When polyglycerol fatty acid ester and/or lipid is used in the coating agent, the coated composition may be manufactured by the process which comprises melting the polyglycerol fatty acid ester and/or lipid together with optional additives by heating, emulsifying the melt with water, spraying the emulsion to the surface of the composition for coating such as matrix and drying. The coated composition also may be manufactured by charging an equipment such as a coating pan with a preheated composition for coating, adding the coating agent to melt and spread over.
   The coating is conducted generally at about 25 ∼ about 60°C, preferably about 25 ∼ about 40°C.
   The coating time can be appropriately selected according to the coating method, characteristics and amount of the coating agent, and characteristics of the composition for coating, among other factors.
   In the prolonged gastrointestinal residence type solid preparation, insofar as a sufficient adhesion to the mucosa in the digestive tract can be assured by said viscogenic agent, the preparation may be optionally further coated with a conventional gastric coating or a water-soluble coating agent.
   Using the fine granules or granules obtained in the above manner, tablets and capsules can be manufactured by the conventional method. The tablets thus manufactured are particularly useful for patients who have difficulties in swallowing, because the tablets, when taken together with water, poorly adhere to the esophagus.
   The sustained-release oral preparation of the present invention may further contain a swelling accelerating agent such as low-substituted hydroxypropyl cellulose and the like. Thus, the "viscogenic agent-containing matrix" and "viscogenic agent-containing coating agent" referred to above may contain a swelling accelerating agent such as low-substituted hydroxypropylcellulose and the like.
   As the low-substituted hydroxypropylcellulose, employed is low-substituted hydroxypropylcellulose having a hydroxypropoxyl content of 5.0∼16.0 weight %.
   Examples of low-substituted hydroxypropylcellulose include LH-11 (hydroxypropoxyl content: 10.0∼13.0 wt. %, particle size: under 149 µ m sieve ≧ 98 wt. %, on 177 µ m sieve ≦ 0.5 wt. %), LH-20 (hydroxypropoxyl content: 13.0∼16.0 wt. %, particle size: under 74 µ m sieve: ≧ 90 wt. %, on 105 µ m sieve ≦ 1.0wt. %), LH-21 (hydroxypropoxyl content: 10.0 ∼ 13.0 wt. %, particle size: under 74 µ m sieve ≧ 90 wt. %, on 105 µ m sieve ≦ 1.0 wt. %), LH-22 (hydroxypropoxyl content: 7.0∼10.0 wt. %, particle size: under 74 µ m sieve ≧ 90 wt. %, on 105 µ m sieve ≦ 1.0 wt. %), and LH-31 (hydroxypropoxyl content: 10.0∼13.0 wt. %, average particle diameter ≦ about 30 µ m).
   The amount of the swelling accelerating agent used, based on the whole sustained-release oral preparation, may for example be about 0.5 ∼ about 50 weight %, preferably about 1 ∼ about 40 weight %, more preferably about 1 ∼ about 30 weight %.
2) The per se known method can be employed for the manufacture of a liquid prolonged gastrointestinal residence type preparation. An exemplary method may comprise adding for dispersion or dissolution the polyglycerol fatty acid ester and/or lipid which is liquid at ambient temperature, viscogenic agent and carbohydrolase inhibitor all simultaneously or each independently. In accordance with a method analogous thereto, a syrup, an emulsion, a suspension or a capsule can be manufactured.

The sustained-release oral preparation of the present invention is low in toxic potential and can be administered safely to mammals (e.g. cat, bovine, dog, horse, goat, monkey, human) requiring the treatment or prophylaxis of diabetes or inhibition of postprandial hyperglycemia. Particularly, the valiolamine derivative for use in the present invention, for example [2-hydroxy-1-(hydroxymethyl)ethyl]valiolamine (voglibose), is very high in safety rating with the acute oral toxicity (LD₅₀) values in mice (NRM1) and rats (Wistar) being 14.7 ∼ 21.5 grams/kilogram body weight (mice) and about 20 grams/kilogram body weight (rats), respectively.

The indications are insulin-dependent diabetes mellitus, non-insulin-dependent diabetes mellitus, insulinemia, insulin receptor abnormality, renal diabetes, and the acute complications (infections etc.) or chronic complications (neuropathy, renal impairment, cardiovascular and cerebrovascular disorders, and dermal and bone complications), among others.

The sustained-release oral preparation of the present invention can be administered orally to mammals inclusive of humans. If desired, pharmacologically and pharmaceutically acceptable additives (e.g. diluent, excipient, binder, disintegrant, coloring agent, stabilizer, etc.) can be used as mentioned hereinbefore.

The dosage of the sustained-release oral preparation according to the present invention varies depending on dosage forms, methods of administration, and kinds of carbohydrolase inhibitors to be administered, but for use in the therapy of diabetes, it is sufficient to administer comparatively very small doses. For example, the dosage of the above valiolamine derivative such as voglibose is usually 0.01 ∼ 100 milligrams/day, preferably 0.05 ∼ 10 mg/day, more preferably 0.1 ∼ 2 mg/day, per adult human, and generally this amount is taken once or in 2∼3 divided doses, preferably before meal.

Particularly, by administering the preparation of this invention at the frequency of only once to about 2 times a day, the postprandial hyperglycemia can be remarkably inhibited as the result of gradual release of the carbohydrolase inhibitor in the small amount, so that the adverse effects caused by elevation of blood glucose can be obviated in diabetic patients.

If necessary, the sustained-release oral preparation of the present invention can be used in combination with other drugs for enhanced efficacy. Such drugs can be either incorporated in the preparation of the invention or administered separately. As such drugs, there can be mentioned antidiabetics other than carbohydrolase inhibitors, such as insulin, insulin-sensitivity enhancers (pioglitazone (hydrochloride), troglitazone, losiglitazone, etc.), sulfonylurea type insulin secretion enhancers (Euglucon, Daonil, Glimicron, Melbin, Dibetos B, Pamilcon, Cymerin, Kinedak, Glimiran, Buformin hydrochloride, Diaglyco, etc.), α₂-antagonists, β ₃-antagonists, etc.; vitamins such as vitamin B₁₂, vitamin B₁, vitamin B₆, etc.; antithrombotic agents; antiulcer agents; antihyperlipemic agents such as anticholesterolemic drugs and triglyceride-lowering agents; prophylactic and therapeutic drugs for atherosclerosis; kampo medicines; prophylactic and therapeutic drugs for nephropathy; prophylactic and therapeutic drugs for cardiomyopathy; and prophylactic and therapeutic drugs for hypertension.

The dosage of these drugs can be appropriately selected with reference to the conventional dosage in clinical use.

In accordance with the present invention, the dosage of the carbohydrolase inhibitor can be reduced as compared with the monotherapy dose. To be specific, the dosage of the carbohydrolase inhibitor is likely to be reduced to one-half through about one-twentieth of the monotherapy dose.

The following examples and experimental examples are further illustrative of the present invention and, yet, by no means limitative of the scope of the invention.

### Example 1

Behenyl hexa(tetra)glyceride (Sakamoto Pharmaceutical Co., Trade name : HB-310), 86.0 g, was weighed out and melted by heating at 84°C. To this melt were added 4 g of voglibose (i.e. [2-hydroxy-1-(hydroxymethyl)ethyl]valiolamine) and 10.0 g of acrylic acid polymer (Wako Pure Chemical Ind. Co., Trade name : HIVISWAKO 104) in this order, which was stirred for dispersion at a constant temperature of 84°C for 15 minutes. The molten mixture was dripped on a 15 cm-diameter aluminum disk revolving at 1950 rpm at a feeding rate of 10 g/min. to provide spherical fine granules which passed a 42-mesh (350 µ m) sieve and remained on a 60-mesh (250 µ m) sieve (hereinafter referred to briefly as 42/60-mesh).

### Example 2

Hydrogenated castor oil (Freund Industrial Co., Ltd., Trade name : Lubriwax 101), 63 g, and behenyl hexa(tetra)glyceride (Sakamoto Pharmaceutical Co., Trade name : HB-310), 5.0 g, were weighed out and melted by heating at 84°C. To the resulting melt were added 4 g of voglibose, 8.0 g of acrylic acid polymer (Wako Pure Chemical Ind. Co., Trade name : HIVISWAKO 104) and 20 g of curdlan (Takeda Chemical Industries, Ltd.) in this order, which was stirred for dispersion at a constant temperature of 84°C for 15 minutes. The molten mixture was then dripped on a 15 cm-diameter aluminum disk revolving at 1950 rpm at a feeding rate of 10 g/min. to provide 42/60-mesh spherical fine granules.

### Example 3

Hydrogenated castor oil (Freund Industrial Co., Ltd., Trade name : Lubriwax 101), 63 g, and behenyl hexa(tetra)glyceride (Sakamoto Pharmaceutical Co., Trade name : HB-310), 5.0 g, were weighed out and melted by heating at 84°C. To this melt were added 4 g of voglibose, 8.0 g of acrylic acid polymer (Wako Pure Chemical Id. Co., Ltd., Trade name : HIVISWAKO 104) and 20 g of low-substituted hydroxypropylcellulose (Shin-Etsu Chemical Co., Ltd., Trade name : L-HPC) in this order, which was stirred for dispersion at a constant temperature of 84°C for 15 minutes. The molten mixture was then dripped on a 15 cm-diameter aluminum disk revolving at 1950 rpm at a feeding rate of 10 g/min. to provide 42/60-mesh spherical fine granules.

### Example 4

Behenyl hexa(tetra)glyceride (Sakamoto Pharmaceutical Co., Trade name : HB-310), 82.5 g, was weighed out and melted by heating at 84°C. To the resulting melt were added 0.5 g of voglibose, 8.0 g of acrylic acid polymer (Wako Pure Chemical Ind. Co., Ltd., Trade name : HIVISWAKO 104), and 10 g of low-substituted hydroxypropylcellulose (Shin-Etsu Chemical Co., Ltd., Trade name : L-HPC) in this order, which was stirred for dispersion at a constant temperature of 84°C for 15 minutes. The molten mixture was then dripped on a 15 cm-diameter aluminum disk revolving at 1950 rpm at a feeding rate of 10 g/min. to provide 42/60-mesh spherical fine granules.

### Example 5

Hydrogenated castor oil (Freund Industrial Co., Ltd., Trade name : Lubriwax 101), 35 g, and behenyl hexa(tetra)glyceride (Sakamoto Pharmaceutical Co., Ltd., Trade name : HB-310), 1.0 g, were weighed out and melted by heating at 84°C. To this melt were added 35 g of voglibose, 5.0 g of acrylic acid polymer (Wako Pure Chemical Ind. Co., Ltd., Trade name : HIVISWAKO 104), and 5.0 g of low-substituted hydroxypropylcellulose (Shin-Etsu Chemical Co., Ltd., Trade name : L-HPC) in this order, which was stirred for dispersion at a constant temperature of 84°C for 15 minutes. The molten mixture was then dripped on a 15 cm-diameter aluminum disk revolving at 1950 rpm at a feeding rate of 10 g/min. to provide 42/60-mesh spherical fine granules.

### Example 6

Carnauba wax (Freund Industrial Co., Ltd., Trade name : Polishing Wax-103), 88.5 g, was weighed out and melted by heating at 95°C. To the resulting melt were added 1.5 g of voglibose and 10.0 g of acrylic acid polymer (Wako Pure Chemical Ind. Co., Ltd., Trade name : HIVISWAKO 104) in this order, which was stirred for dispersion at a constant temperature of 95°C for 15 minutes. This molten mixture was dripped on a 15 cm-diameter aluminum disk revolving at 1950 rpm at a feeding rate of 10 g/min. to provide 42/60-mesh spherical fine granules.

### Example 7

Hydrogenated castor oil (Freund Industrial Co., Ltd., Trade name : Lubriwax 101), 89.75 g, was weighed out and melted by heating at 84°C. To the resulting melt were added 0.25 g of voglibose, 10 g of acrylic acid polymer (Wako Pure Chemical Ind. Co., Ltd., Trade name : HIVISWAKO 104), and 5.0 g of low-substituted hydroxypropylcellulose (Shin-Etsu Chemical Co., Ltd., Trade name : L-HPC) in this order, which was stirred for dispersion at a constant temperature of 84°C for 15 minutes. The molten mixture was then dripped on a 15 cm-diameter aluminum disk revolving at 1950 rpm at a feeding rate of 10 g/min. to provide 42/60-mesh spherical fine granules.

### Example 8

A mixed powder consisting of 108.7 g of lactose, 46.6 g of corn starch, 28.5 g of croscarmellose sodium (FMC Corporation, Trade name : Ac-Di-Sol), 5.7 g of hydroxypropylcellulose (Nippon Soda Co., Ltd., Trade name : HPC-L) and 0.5 g of magnesium stearate was admixed with 10 g of the fine granules obtained in Example 1. Of the mixture thus obtained, a 225 mg portion was tabletted with a 8.5 mm-diameter flat punch at a tabletting pressure of 0.5 ton/cm² to provide tablets.

### Example 9

A mixed powder consisting of 37.2 g of lactose, 16.0 g of corn starch, 9.7 g of croscarmellose sodium (FMC Corporation, Trade name : Ac-Di-Sol), 0.9 g of hydroxypropylcellulose (Nippon Soda Co., Ltd., Trade name : HPC-L) and 0.2 g of magnesium stearate was admixed with 10 g of the fine granules obtained in Example 6. Of the mixture thus obtained, a 225 mg portion was tabletted with a 8.5 mm-diameter flat punch at a tabletting pressure of 0.5 ton/cm² to provide tablets.

### Comparative Example

Voglibose was dissolved in distilled water to prepare a 0.1 mg/ml aqueous solution of voglibose.

### Experimental Example

To SD rats (male, 7 weeks old), fasted for 20 hours before administration, the fine granules obtained in Example 7 or the aqueous solution obtained in Comparative Example was administered at a dose of 0.5 mg/kg body weight as voglibose.

Immediately after administration, the rats were loaded with 1.0 g/kg body wt. of water-soluble starch and after an interval of 4 hours, similarly reloaded.

Before loading with the water-soluble starch and 30 minutes after the loading, blood was taken from the tail vein of rats and the plasma glucose concentration was measured.

Since the peak of blood glucose level occurred at 30 minutes after loading with water-soluble starch, the amount of increase in blood glucose during the period from starch loading to 30 minutes after loading was calculated and used as the indicator of efficacy.

The results are shown in Table 1.

**Table 1**

| | Elevation of blood glucose (mg/dl) |
|---|---|
| Invention - voglibose fine granules | 38±6.2 |
| Control - aq. solution of voglibose | 66±6.3 |
| (mean ± S.D., n=5) | |

Although no difference was found between the invention group and the control group in the amount of increase in blood glucose at the time immediately after voglibose administration, a definite inhibition of the elevation of blood glucose was found after the loading with water-soluble starch at 4 hours after administration in the invention group as compared with the control group, indicating the superiority of the sustained-release preparation of the present invention.

### INDUSTRIAL APPLICABILITY

The sustained-release oral preparation of the present invention stays long after oral administration within the digestive tract of mammals, by adhesion and in other way, and releases a carbohydrolase inhibitor, such as an α-glucosidase inhibitor, gradually and continuously over long time. The carbohydrolase inhibitor comes into contact with the carbohydrolase in the digestive tract to inhibit decomposition of carbohydrates and, thus, provides prophylactic and therapeutic efficacies in diabetes, such as inhibition of postprandial hyperglycemia.

## Claims

1. A sustained-release oral preparation comprising a carbohydrolase inhibitor.

2. The preparation as defined in Claim 1, wherein the sustained-release oral preparation is a prolonged gastrointestinal residence type preparation.

3. The preparation as defined in Claim 2, which is a prolonged upper digestive tract residence type preparation.

4. The preparation as defined in Claim 1, wherein the carbohydrolase inhibitor is an α-glucosidase inhibitor.

5. The preparation as defined in Claim 1, which is gastrointestinal mucosa-adherent.

6. The preparation as defined in Claim 1, which comprises a matrix comprising a polyglycerol fatty acid ester and/or a lipid.

7. The preparation as defined in Claim 6, which further comprises a substance which develops viscosity in response to water.

8. The preparation as defined in Claim 6, wherein the carbohydrolase inhibitor is contained in the matrix.

9. The preparation as defined in Claim 7, wherein the substance which develops viscosity in response to water is contained in the matrix.

10. The preparation as defined in Claim 7, wherein the matrix is coated with the substance which develops viscosity in response to water.

11. The preparation as defined in Claim 1, wherein the carbohydrolase inhibitor is voglibose.

12. The preparation as defined in Claim 1, which is for antidiabetics.

13. The preparation as defined in Claim 1, which is a solid preparation.

14. The preparation as defined in Claim 13, wherein the solid preparation is in the form of fine granules or granules.

15. The preparation as defined in Claim 1, wherein the carbohydrolase inhibitor is contained in a proportion of about 0.05 ∼ about 5.0 weight % based on the whole preparation.

16. The preparation as defined in Claim 6, wherein the polyglycerol fatty acid ester has a molecular weight of about 200 ∼ about 5000.

17. The preparation as defined in Claim 6, wherein the polyglycerol fatty acid ester has a hydrophile-lipophile balance number of 1∼15.

18. The preparation as defined in Claim 6, wherein the polyglycerol fatty acid ester is used in a proportion of about 0.01 ∼ about 10000 weight parts per weight part of the carbohydrolase inhibitor;

19. The preparation as defined in Claim 7, wherein the substance which develops viscosity in response to water is an acrylic acid polymer or a salt thereof.

20. The preparation as defined in Claim 7, wherein the substance which develops viscosity in response to water is contained in a proportion of about 0.05 ∼ about 99 weight % based on the whole matrix.
